# EUROPEAN PATENT APPLICATION

(11) **EP 4 670 754 A1**
(43) Date of publication of application: **31.12.2025**
(21) Application number: 23923906.4
(22) Date of filing: 28.11.2023
(51) Int. Cl.: A61M 1/34

(54) **APHERESIS FILTER AND METHOD FOR ELIMINATING RESIDUAL PLATINUM IN BLOOD**

(30) Priority: 22.02.2023 ES 202330132
(71) Applicant: BYOTIC R&D SERVICES SOCIEDAD LIMITADA UNIPERSONAL, 17005 Girona (ES)
(72) Inventor: MOYANO CUDINACH, Manel, 17005 Girona (ES); SERAS SEVA, Laura, 17005 Girona (ES); SERRANO MORILLAS, Natalia, 17005 Girona (ES)
(74) Representative: Araujo, Daniel
(86) International application number: PCT/ES2023/070703
(87) International publication number: WO 2024/175813

(57) **Abstract**

The invention relates to a therapeutic apheresis filter for eliminating heavy metals in blood and especially residual platinum in cancer patients after chemotherapy treatment. The filter comprises a casing (1) that can be coupled to an extracorporeal apheresis device and that has inlet and outlet openings (11a, 12a) for circulating therein blood or plasma extracted from a patient and that delimits a cavity containing a biomaterial (3), made up of granulated or powdered egg membrane particles for retaining the heavy metal molecules inside the filter by adsorption. The invention further comprises a method using the apheresis filter with egg membrane as a biomaterial to eliminate heavy metals in blood.

## Description

### Technical Field

The present invention relates to a therapeutic apheresis filter for eliminating residual platinum in blood, in cancer patients after chemotherapy treatment.

### Background

The clinical practice known as therapeutic apheresis is now widely known and used, which involves removing one or more elements from the blood by passing the blood or plasma from the blood collected from a patient through an adsorbent filter contained in an extra-corporeal equipment, itself known, and returning the blood to the patient. Apheresis is therefore a closed-loop process in which plasma is continuously restored to blood cells and re-enters the patient after having been treated outside the patient.

Extracorporeal equipment used in apheresis generally allows filters of various standardized sizes. Basically comprises an outer casing provided with an inlet and an outlet for the circulation of blood through a specific product suitable for retaining, and thus reducing the quantity in the blood, of certain molecules; for example, a specific element, for example LDL, viral particles, or immunoglobulins, among many others.

For example, WO2012142180A1 discloses a method for removing pathogens from a patient's blood by separating plasma from extracorporeal circulating blood, inactivating the pathogen by physical means, and returning the blood to the patient. This method focuses on removing circulating tumour cells in blood after treatment or removal of a tumour and the elimination of antibodies causing autoimmune diseases.

One of the current challenges is the elimination of heavy metals in the blood; although there are certain chelators that can block the negative effect of some metals, there is no known technique on the market that can reduce heavy metals in the blood by therapeutic apheresis.

This is a problem because after chemotherapy cancer treatments, there is an accumulation of heavy metals, particularly platinum, in the blood, which can cause damage to the body.

The technical problem that arises is therefore the development of a filter applicable in therapeutic apheresis and a method with technical characteristics that allow the elimination of residual platinum in the blood of cancer patients after chemotherapy treatment and that does not involve the use of other molecules or drugs that can worsen the functioning of essential organs such as the kidney or liver.

CN1958465A discloses a method for treating industrial and domestic urban wastewater and extracting precious metals by using biological waste egg membranes.

The applicant of the invention is unaware of the existence of any therapeutic apheresis filters intended for the reduction of residual platinum in blood or plasma, using the filter material of the present invention.

### Explanation of the invention

The therapeutic apheresis filter subject of the invention has technical characteristics suitable for solving in a simple way the problem described above. Which is the reduction in blood of residual platinum from oncological treatments with chemotherapy, with the intention of reducing the toxic side effects of platinum in the long term, from an extracorporeal process such as therapeutic apheresis.

The filter of the invention is of the type comprising a casing of suitable dimensions for use in extracorporeal apheresis device and which has inlet and outlet openings for the circulation of blood, or plasma, collected from a patient, through a cavity delimited by said casing, before being returned to said patient. According to the invention, this apheresis filter comprises a biomaterial inside the casing made up of egg membrane particles, in granulated or powdered form, which by means of an ion exchange, creates a bond that favours the adsorption union between the platinum molecule and the egg membrane particles making up the biomaterial, and the retention of platinum molecules inside the filter, preventing their return to the patient's bloodstream.

Studies have shown that egg membrane powder has a higher affinity for platinum than other heavy metals, making it particularly suitable for use as a biomaterial for eliminating residual platinum from chemotherapy treatments in cancer patients.

Advantageously, the egg membrane, in granulated or powdered form, arranged inside the casing, is housed in a macroparticle filtration membrane that allows the flow between pores of the main proteins and plasma molecules. To allow this passage, the filtration membrane has a pore size equal to or larger than 100 nm.

The egg membrane, granulated or powdered, in addition to the affinity with platinum molecules indicated above, has characteristics that make it especially suitable for the proposed use, namely, it provides a large adsorption area, is biocompatible, complies with regulations for clinical use and is easily industrialisable.

This invention also includes a method for eliminating platinum from blood by means of therapeutic apheresis and the use of the filter described above in an extracorporeal apheresis device.

The method comprises: a) collecting blood from a patient, b) passing the blood, or blood plasma collected from the patient, through a filter membrane and a biomaterial made up of egg membrane particles, in granulated or powdered form, contained in the apheresis filter placed in an extracorporeal apheresis device, c) retention by adsorption of the platinum molecules by the egg membrane particles inside the apheresis filter and d) returning the treated blood or plasma to said patient.

### Brief description of the content of the drawings.

To complement the description being given and in order to facilitate understanding of the features of the invention, this description is accompanied by a set of drawings in which, for illustrative and non-limiting purposes, the following has been represented:

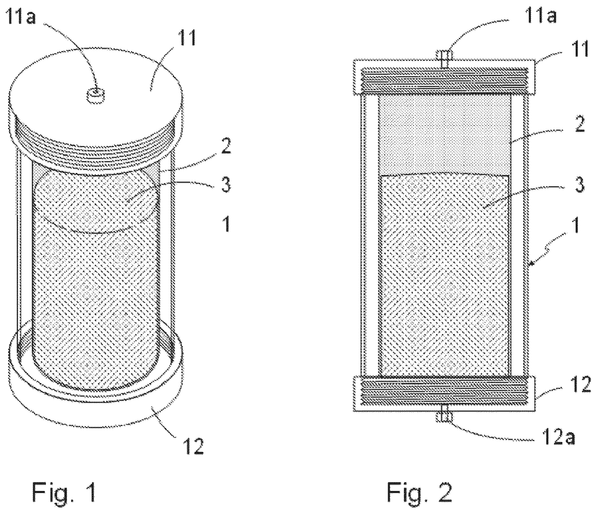

- Figure 1 shows a perspective view of an embodiment of the apheresis filter for the separation of heavy metals in blood.
- Figure 2 shows an elevation view of the apheresis filter in the previous figure.

### Detailed description of embodiments of the invention.

In the example shown in the figures, the apheresis filter comprises a casing (1) made of plastic material and dimensions suitable for use in extracorporeal apheresis device (not shown), provided in this case with two threaded caps (11, 12) and two inlet and outlet openings (11a, 12a) for the circulation through its interior of blood or plasma collected from a patient.

Said casing (1) delimits a cavity in which is housed a macroparticle filtration membrane (2) containing a biocompatible material (3) made up of granulated or powdered egg membrane particles, which by adsorption retains the residual platinum contained in the blood or plasma inside the filter; the blood or plasma then returns to the circulatory flow of said patient.

As indicated, this filter is mainly applicable in the elimination of residual platinum in cancer patients after undergoing chemotherapy treatment.

According to the invention, the pore size of the filter membrane is at least 100 nm to allow the passage through the same of the main proteins and plasma molecules and to retain inside said membrane the egg membrane particles in charge of retaining the platinum molecules. inside said membrane.

Having sufficiently described the nature of the invention, as well as a preferred embodiment, it is noted for the appropriate purposes that the materials, shape, size and arrangement of the elements described may be modified, provided that this does not entail an alteration of the essential characteristics of the invention claimed below.

## Claims

1. A therapeutic apheresis filter for eliminating residual platinum in blood in cancer patients after chemotherapy treatment; comprising a casing (1) that can be coupled to extracorporeal apheresis device and having inlet and outlet openings (11a, 12a) for circulating therein blood or plasma extracted from a patient and delimiting a cavity containing a biomaterial (3); **characterized in that** said casing (1) comprises two threaded caps (11, 12), a macroparticle filtration membrane (2) containing the biomaterial (3) being housed inside it, said biomaterial (3) being made up of granulated or powdered egg membrane particles, which by means of an ion exchange creates a bond that favours the adsorption union between the platinum molecules and the egg membrane particles making up the biomaterial (3), and the retention of said platinum molecules inside the filter.

2. The apheresis filter of claim 1, wherein the filtration membrane has a pore size of at least 100 nm, suitable for passage of major proteins and molecules.

3. Method for eliminating residual platinum in blood, especially residual platinum in cancer patients after chemotherapy treatment, by means of therapeutic apheresis in an extracorporeal apheresis device provided with the apheresis filter of the preceding claims; **characterized in that** it comprises:
a) collecting blood from a patient,
b) passing the blood, or blood plasma collected, through a filter membrane and a biomaterial made up of egg membrane particles, in granulated or powdered form, contained in the apheresis filter in an extracorporeal apheresis device,
c) retention by adsorption of the platinum molecules by the egg membrane particles inside the apheresis filter and
d) returning the treated blood or plasma to said patient.
